# EUROPEAN PATENT APPLICATION

(11) **EP 3 922 720 A1**
(43) Date of publication of application: **15.12.2021**
(21) Application number: 20382498.2
(22) Date of filing: 09.06.2020
(51) Int. Cl.: C12N 15/113, C07K 14/47, A61P 9/00

(54) **THERAPY TO PREVENT ADVERSE CARDIAC REMODELING FOLLOWING AN ACUTE MYOCARDIAL INFARCTION**

(71) Applicant: Universidad de Murcia, 30072 Murcia (ES)
(72) Inventor: PASCUAL FIGAL, Domingo Andrés, 30072 Murcia (ES); LAX PÉREZ, Antonio Manuel, 30072 Murcia (ES); ASENSIO LÓPEZ, María Carmen, 30072 Murcia (ES)
(74) Representative: Hoffmann Eitle

(57) **Abstract**

The present invention refers to a composition comprising a compound capable of reducing the expression of the Yin Yang 1 (Yy1) gene in cardiac cells of a human or animal subject with respect to the expression observed in the absence of the compound in said cells, wherein the compound is an interference RNA (RNAi) of the Yy1 gene, and wherein said compound is for use in the treatment of myocardial fibrosis, cardiac inflammation, cardiac hypertrophy and/or functional impairment 2 to 48 hours after acute myocardial infarction (AMI).

SEQUENCE LISTING

<110> Universidad de Murcia

<120> Therapy to prevent adverse cardiac remodeling following an acute
myocardial infarction

<130> 905 043

<140> EP20382498.2
<141> 2020-06-09

<160> 48

<170> BiSSAP 1.3.6

<210> 1
<211> 21
<212> RNA
<213> Artificial Sequence

<220>
<223> siRNA (1) sense sequence

<400> 1
cuguugucca gaauacuuau u                                                21

<210> 2
<211> 21
<212> RNA
<213> Artificial Sequence

<220>
<223> siRNA (1) antisense sequence

<400> 2
uaaguauucu ggacaacagu u                                                21

<210> 3
<211> 21
<212> RNA
<213> Artificial Sequence

<220>
<223> siRNA (2) sense sequence

<400> 3
cauguagaau caaauauuau u                                                21

<210> 4
<211> 21
<212> RNA
<213> Artificial Sequence

<220>
<223> siRNA (2) antisense sequence

<400> 4
uaauauuuga uucuacaugu u                                                21

<210> 5
<211> 21
<212> RNA
<213> Artificial Sequence

<220>
<223> siRNA (3) sense sequence

<400> 5
gcuccaagaa caauagcuuu u                                                21

<210> 6
<211> 21
<212> RNA
<213> Artificial Sequence

<220>
<223> siRNA (3) antisense sequence

<400> 6
aagcuauugu ucuuggagcu u                                                21

<210> 7
<211> 21
<212> RNA
<213> Artificial Sequence

<220>
<223> siRNA (4) sense sequence

<400> 7
caacuaaccu gaaaucucau u                                                21

<210> 8
<211> 21
<212> RNA
<213> Artificial Sequence

<220>
<223> siRNA (4) antisense sequence

<400> 8
ugagauuuca gguuaguugu u                                                21

<210> 9
<211> 21
<212> RNA
<213> Artificial Sequence

<220>
<223> non-targeting Accell siRNA sense sequences (siCtrl)

<400> 9
uagcgacuaa acacaucaau u                                                21


<210> 10
<211> 21
<212> RNA
<213> Artificial Sequence


<220>
<223> non-targeting Accell siRNA antisense sequences (siCtrl)

<400> 10
uugauguguu uagucgcuau u                                                21


<210> 11
<211> 20
<212> DNA
<213> Artificial Sequence


<220>
<223> BNP-F

<400> 11
cgtcttggcc ttttggcttc                                                  20


<210> 12
<211> 23
<212> DNA
<213> Artificial Sequence


<220>
<223> BNP-R

<400> 12
ggtggtctag caggttcttg aaa                                              23


<210> 13
<211> 19
<212> DNA
<213> Artificial Sequence


<220>
<223> Col1a1-F

<400> 13
ctggcaagaa gggagatga                                                   19


<210> 14
<211> 20
<212> DNA
<213> Artificial Sequence

<220>
<223> Col1a1-R

<400> 14
caccatccaa accactgaaa                                                    20


<210> 15
<211> 24
<212> DNA
<213> Artificial Sequence


<220>
<223> Col3a1-F

<400> 15
acagcaaatt cacttacaca gttc                                               24


<210> 16
<211> 19
<212> DNA
<213> Artificial Sequence


<220>
<223> Col3a1-R

<400> 16
ctcattgcct tgcgtgttt                                                     19


<210> 17
<211> 25
<212> DNA
<213> Artificial Sequence


<220>
<223> CRP-F

<400> 17
gaactttcag ccgaatacat ctttt                                              25


<210> 18
<211> 21
<212> DNA
<213> Artificial Sequence


<220>
<223> CRP-R

<400> 18
ccttcctcga catgtctgtc t                                                  21


<210> 19
<211> 22

<212> DNA
<213> Artificial Sequence

<220>
<223> IL-33-F

<400> 19
cagaatcatc gagaaaaggc gg                                                22

<210> 20
<211> 20
<212> DNA
<213> Artificial Sequence

<220>
<223> IL-33-R

<400> 20
gccggggaaa tcttggagtt                                                   20

<210> 21
<211> 20
<212> DNA
<213> Artificial Sequence

<220>
<223> IL-6-F

<400> 21
cacttcacaa gtcggaggct                                                   20

<210> 22
<211> 22
<212> DNA
<213> Artificial Sequence

<220>
<223> IL-6-R

<400> 22
tgccattgca caactctttt ct                                                22

<210> 23
<211> 20
<212> DNA
<213> Artificial Sequence

<220>
<223> Myh6-F

<400> 23
accaacctgt ccaagttccg                                                   20

<210> 24
<211> 20
<212> DNA
<213> Artificial Sequence

<220>
<223> Myh6-R

<400> 24
gtcgtgcatc ttcttggcac                                                    20

<210> 25
<211> 20
<212> DNA
<213> Artificial Sequence

<220>
<223> Myh7-F

<400> 25
cgcatcaagg agctcaccta                                                    20

<210> 26
<211> 20
<212> DNA
<213> Artificial Sequence

<220>
<223> Myh7-R

<400> 26
cttggacagg ttggtgttgg                                                    20

<210> 27
<211> 22
<212> DNA
<213> Artificial Sequence

<220>
<223> MyO-F

<400> 27
catggttgca ccgtgctcac ag                                                 22

<210> 28
<211> 20
<212> DNA
<213> Artificial Sequence

<220>
<223> MyO-R

<400> 28
gagcccatgg ctcagccctg                                                    20

<210> 29
<211> 20
<212> DNA
<213> Artificial Sequence

<220>
<223> Nppa-F

<400> 29
gcttccaggc catattggag                                                    20

<210> 30
<211> 19
<212> DNA
<213> Artificial Sequence

<220>
<223> Nppa-R

<400> 30
gggggcatga cctcatctt                                                     19

<210> 31
<211> 21
<212> DNA
<213> Artificial Sequence

<220>
<223> PTX3-F

<400> 31
caggatgcac gcttccaaaa a                                                  21

<210> 32
<211> 19
<212> DNA
<213> Artificial Sequence

<220>
<223> PTX3-R

<400> 32
tgcccgcagg ttgtgaaac                                                     19

<210> 33
<211> 22
<212> DNA
<213> Artificial Sequence

<220>
<223> Smad2-F

<400> 33
aagccatcac cactcagaat tg                                                22

<210> 34
<211> 23
<212> DNA
<213> Artificial Sequence

<220>
<223> Smad2-R

<400> 34
cactgatcta ccgtatttgc tgt                                               23

<210> 35
<211> 21
<212> DNA
<213> Artificial Sequence

<220>
<223> Smad3-F

<400> 35
gaggagaagt ggtgcgagaa g                                                 21

<210> 36
<211> 21
<212> DNA
<213> Artificial Sequence

<220>
<223> Smad3-R

<400> 36
atgcacttgg tgttcacgtt c                                                 21

<210> 37
<211> 24
<212> DNA
<213> Artificial Sequence

<220>
<223> sST2-F

<400> 37
gctaggacct ctggctaatg tatc                                              24

<210> 38
<211> 20
<212> DNA
<213> Artificial Sequence

<220>

<223> sST2-R

<400> 38
atggtgtgtt cactaggcgg                                                    20


<210> 39
<211> 20
<212> DNA
<213> Artificial Sequence


<220>
<223> ST2L-F

<400> 39
tgggcaaggt aaaccgactg                                                    20


<210> 40
<211> 20
<212> DNA
<213> Artificial Sequence


<220>
<223> ST2L-R

<400> 40
   caccccctcc tcactaccta                                                     20


<210> 41
<211> 20
<212> DNA
<213> Artificial Sequence


<220>
<223> TGF-b-F

<400> 41
caacccaggt ccttcctaaa                                                    20


<210> 42
<211> 20
<212> DNA
<213> Artificial Sequence


<220>
<223> TGF-b-R

<400> 42
ggagagccct ggataccaac                                                    20


<210> 43
<211> 20
<212> DNA
<213> Artificial Sequence

<220>
<223> TNF-a-F

<400> 43
agttctatgg cccagaccct                                                    20


<210> 44
<211> 19
<212> DNA
<213> Artificial Sequence


<220>
<223> TNF-a-R

<400> 44
ggtggtttgc tacgacgtg                                                     19


<210> 45
<211> 20
<212> DNA
<213> Artificial Sequence


<220>
<223> Yy1-F

<400> 45
tgagaaagca tctgcacacc                                                    20


<210> 46
<211> 20
<212> DNA
<213> Artificial Sequence


<220>
<223> Yy1-R

<400> 46
cgcaaattga agtccagtga                                                    20


<210> 47
<211> 23
<212> DNA
<213> Artificial Sequence


<220>
<223> a-SMA-F

<400> 47
gtgctgtccc tctatgcctc tgg                                                23


<210> 48
<211> 23
<212> DNA

<213> Artificial Sequence

<220>
<223> a-SMA-R

<400> 48
ggcacgttgt gagtcacacc atc 23

## Description

### Technical field of the invention

The present invention refers to the biomedical field, in particular to the reduction of sST2 production by inhibiting Yy1 factor for preventing pathological myocardial remodeling after myocardial infarction and left ventricular dysfunction.

### Background of the invention

In patients suffering an acute myocardial infarction (AMI), opening the occluded coronary artery is the best strategy to recover oxygen supply and to limit ischemic myocardial damage. However, the reperfusion therapy based on primary angioplasty and/or fibrinolytics, is effective only when performed within the first 1-2 hours after symptoms onset [1]. Unfortunately, a significant portion of patients does not access to the reperfusion therapy in a timely manner, and every 30 min of delay is associated with a relative increase of 7.5% in death risk at one year [2]. This excess of mortality is explained because ischemic time duration is a major determinant of infarct size, which leads to pathological changes in cardiac function and structure. These pathological processes are known as adverse cardiac remodeling and include contractile dysfunction, chamber dilatation and myocardial abnormalities [3, 4]. The extension of these processes determines the progression to heart failure (HF), ventricular arrhythmias and death in the short- and long-term follow-up. Therefore, the search for new therapies in order to minimize adverse myocardial remodeling and cardiac complications following AMI are a priority in cardiovascular medicine.

Soluble suppression of tumorigenesis 2 (sST2) is a member of the interleukin 1 receptor family, also known as interleukin 1 receptor-like 1 (IL1RL1)[5] with two main isoforms: a membrane-bound receptor (ST2 ligand [ST2L]) and a soluble ST2 isoform (sST2)[4]. sST2 is a unique biomarker associated with pathological cardiac processes [6,7]. In particular, in patients suffering an acute AMI, circulating concentrations of sST2 have repeatedly identify a higher risk of death and the progression of adverse myocardial remodeling to HF in the short-term (30 days) and also in the long-term follow-up [8-13].

Interleukin-33 (IL-33), by interacting with ST2L [14] triggers a cardioprotective, anti-remodeling response [7, 15] that is associated with the blocking of both IκBα phosphorylation as well as the activation of NF-κB promoter activity [16]. Increased concentrations of sST2 in the circulation can bind to IL-33 directly and act as a decoy receptor, by inhibiting its binding to membrane-bound ST2L, thus blocking the cardioprotective effects of IL-33; such inhibition results in cardiac hypertrophy, myocardial fibrosis, and ventricular dysfunction [7, 15] . Several experimental studies, have shown that the expression of IL-33 and sST2 in both cardiac fibroblasts and cardiomyocytes increased in response to cardiac stress. Weingberg et al., in 2002, determined that ST2 was the most highly induced transcript in response to biomechanical stress, and the sST2 and ST2L forms were induced in neonatal cardiomyocytes subjected to cyclic strain [10]. Accordingly, using a myocardial infarct model we have shown that an increase in myocardium sST2 levels after 4 weeks positively correlates with cardiac remodeling markers, such as inflammatory and fibrosis markers [17]. Therefore, to favorably regulate the IL-33/ST2 pathway has been suggested as a therapeutic option [18].

Although we know that soluble and membrane forms of ST2 are expressed through cellular-specific modulation of a dual promoter [10], the molecular elements related to the specific sST2 expression following AMI are unknown. Using computational genomics and neonatal cardiomyocytes under biomechanical strain, we identified Yin yang-1 (Yyl) as a transcription factor related to cardiac expression of sST2 [19]. In this *in vitro* study, the silencing of the endogenous Yy1 expression resulted in a decrease in the expression and release of sST2 [19]. Altogether, these findings support the idea that reducing Yy1 expression levels would be a promising anti-adverse cardiac remodeling therapy following AMI.

The present invention provides a new therapy to diminish infarct size and prevent cardiac dysfunction and adverse myocardial remodeling following AMI.

### References

1. Ibanez B, James S, Agewall S, Antunes MJ, Bucciarelli-Ducci C, Bueno H, Caforio ALP, Crea F, Goudevenos JA, Halvorsen S, Hindricks G, Kastrati A, Lenzen MJ, Prescott E, Roffi M, Valgimigli M, Varenhorst C, Vranckx P, Widimský P, Baumbach A, Bugiardini R, Coman IM, Delgado V, Fitzsimons D, Gaemperli O, Gershlick AH, Gielen S, Harjola VP, Katus HA, Knuuti J, et al. 2017 ESC Guidelines for the management of acute myocardial infarction in patients presenting with ST-segment elevation. Eur Heart J 2018;39:119-177.
2. De Luca G, Suryapranata H, Ottervanger J P.et al Time delay to treatment and mortality in primary angioplasty for acute myocardial infarction: every minute of delay counts. Circulation 2004;109:1223-1225.
3. Sutton MG, Sharpe N. Left ventricular remodeling after myocardial infarction: Pathophysiology and therapy. Circulation 2000;101:2981-2988.
4. Konstam MA, Kramer DG, Patel AR, Maron MS, Udelson JE. Left ventricular remodeling in heart failure: Current concepts in clinical significance and assessment. JACC Cardiovasc Imaging 2011;4:98-108.
5. Garlanda C, Dinarello CA, Mantovani A. The interleukin-1 family: back to the future. Immunity 2013;39:1003-18.
6. Pascual-Figal DA, Januzzi JL. The Biology of ST2: The International ST2 Consensus Panel. Am. J. Cardiol. 2015;115:3B-7B.
7. Pascual-Figal DA, Lax A, Perez-Martinez MT, et al. Clinical relevance of sST2 in cardiac diseases. Clin Chem Lab Med 2016;54:29-35.
8. Weir RAP, Miller AM, Murphy GEJ, et al. Serum soluble ST2: a potential novel mediator in left ventricular and infarct remodeling after acute myocardial infarction. J Am Coll Cardiol 2010;55:243-50.
9. Bière L, Garcia G, Guillou S, et al. ST2 as a predictor of late ventricular remodeling after myocardial infarction. Int J Cardiol 2018;259:40-42.
10. Weinberg EO, Shimpo M, De Keulenaer GW, et al. Expression and regulation of ST2, an interleukin-1 receptor family member, in cardiomyocytes and myocardial infarction. Circulation 2002;106:2961-6.
11. Sabatine MS, Morrow DA, Higgins LJ, et al. Complementary roles for biomarkers of biomechanical strain ST2 and N-terminal prohormone B-type natriuretic peptide in patients with ST-elevation myocardial infarction. Circulation 2008;117:1936-44.
12. Shimpo M, Morrow DA, Weinberg EO, et al. Serum levels of the interleukin-1 receptor family member ST2 predict mortality and clinical outcome in acute myocardial infarction. Circulation 2004;109:2186-90.
13. Jenkins WS, Roger VL, Jaffe AS, et al. Prognostic Value of Soluble ST2 After Myocardial Infarction: A Community Perspective. Am J Med 2017;130:1112.e9-1112.e15.
14. Schmitz J, Owyang A, Oldham E, et al. IL-33, an interleukin-1-like cytokine that signals via the IL-1 receptor-related protein ST2 and induces T helper type 2-associated cytokines. Immunity 2005;23:479-90.
15. Seki K, Sanada S, Kudinova AY, et al. Interleukin-33 Prevents Apoptosis and Improves Survival After Experimental Myocardial Infarction Through ST2 Signaling. Circ. Hear. Fail. 2009;2:684-691.
16. Sanada S, Hakuno D, Higgins LJ, Schreiter ER, McKenzie ANJ, Lee RT. IL-33 and ST2 comprise a critical biomechanically induced and cardioprotective signaling system. J. Clin. Invest. 2007;117:1538-49.
17. Sanchez-Mas J, Lax A, Asensio-Lopez M, et al. Modulation of IL-33/ST2 system in post-infarction heart failure: correlation with cardiac remodeling markers. Eur. J. Clin. Invest. 2014;44:643-51.
18. Kakkar R, Lee RT. The IL-33/ST2 pathway: therapeutic target and novel biomarker. Nat Rev Drug Discov 2008;7:827-40.
19. Asensio-Lopez MC, Lax A, Fernandez del Palacio MJ, et al. Yin-Yang 1 transcription factor modulates ST2 expression during adverse cardiac remodeling post-myocardial infarction. J. Mol. Cell. Cardiol. 2019;130:216-233.

### Brief description of the figures

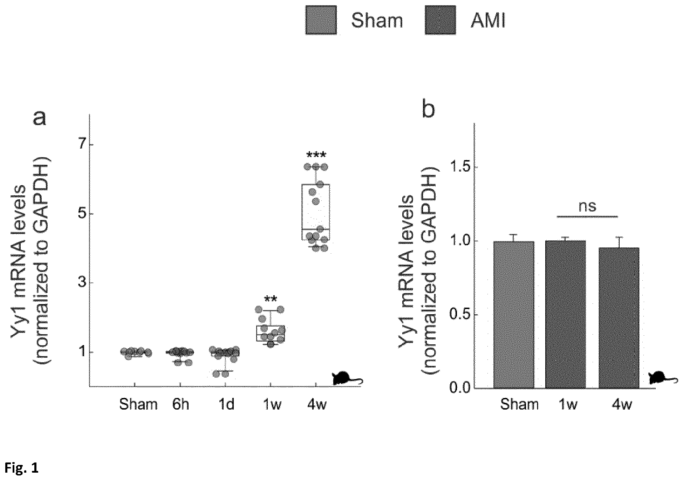

Figure 1.- Yy1 expression levels increases in the infarcted LV area. (a) Yy1 mRNA levels; data were normalized to GAPDH mRNA levels. (b) Yy1 mRNA levels in the remote LV area; data were normalized to GAPDH mRNA levels. ** p<0.01, *** p<0.001 in compared to sham group. ns: non-significant.

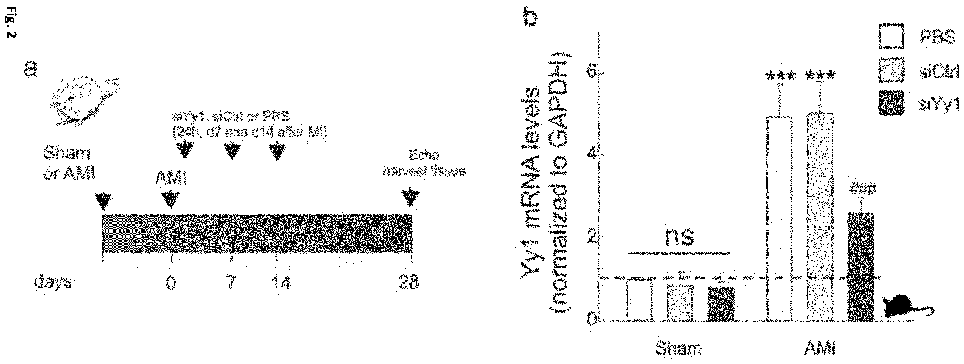

Figure 2.- siYy1 therapy prevents the up-regulation of cardiac Yy1 transcription factor in infarcted LV area. (a) Representative scheme experimental procedure. (b) Yy1 mRNA levels; data were normalized respect to GAPDH mRNA levels. *** p<0.001 respect to their respective sham groups; ### p<0.001 respect to AMI+siCtrl group. siCtrl: interference RNA control; siYy1: specific interference ARN against Yy1 transcription factor; AMI: acute myocardial infarction; ns: non-significant; Yy1: Yin yang-1 transcription factor.

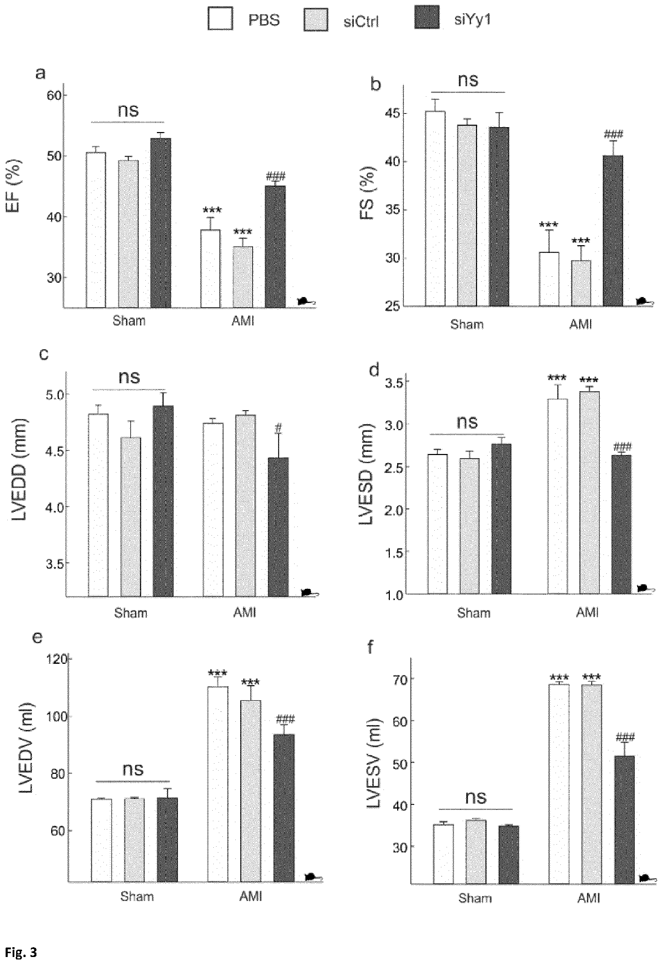

Figure 3.- siYy1 therapy improves cardiac function following AMI. (a-f) Echocardiographic determined EF (a), FS (b), LVEDD (c), LVESD (d), LVEDV (e), or LVESV (f), respectively. *p<0.05, *** p<0.001 respect to their respective sham groups; ### p<0.001 respect to AMI+siCtrl group. EF: ejection fraction; FS: fractional shorting; LVEDD: left ventricular end diastolic dimension; LVESD: left ventricular end systolic dimension; LVEDV: Left ventricular end diastolic volume; LVESV: Left ventricular end systolic volume; other abbreviations are shown above.

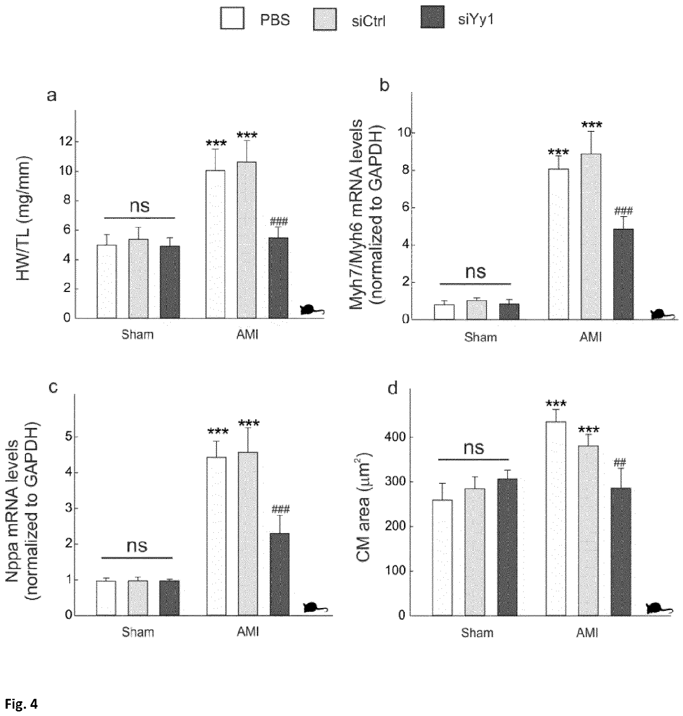

Figure 4.- siYy1 therapy modulates IL-33/ST2L axis. (a-c) IL-33, ST2L and sST2 mRNA levels; data are normalized respect to GAPDH mRNA levels. *** p<0.001 respect to their respective controls; ### p<0.001 respect to AMI+siCtrl group. IL: interleukin; ST2L: membrane receptor ST2L; sST2: soluble isoform ST2.

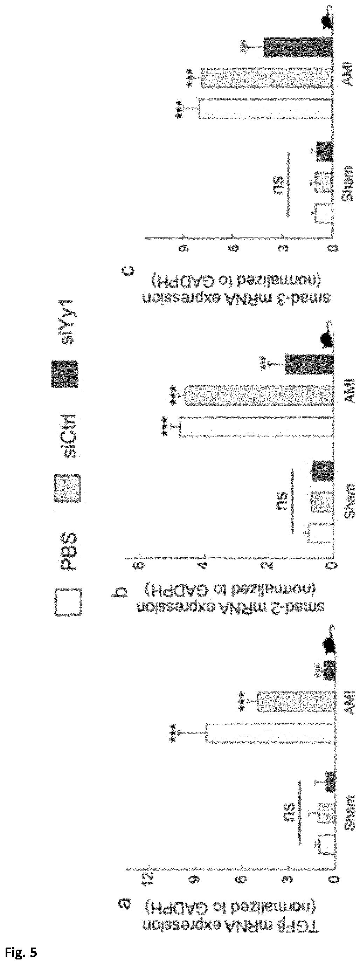

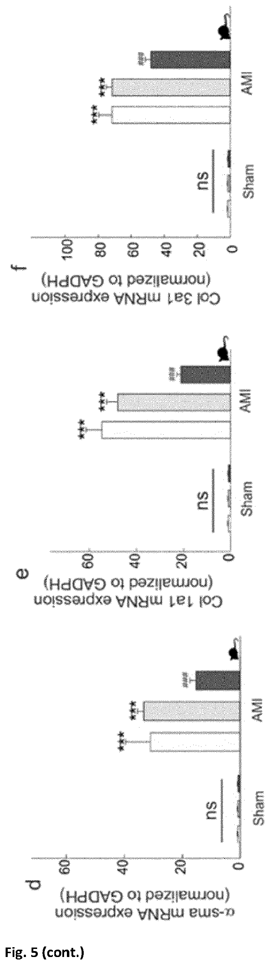

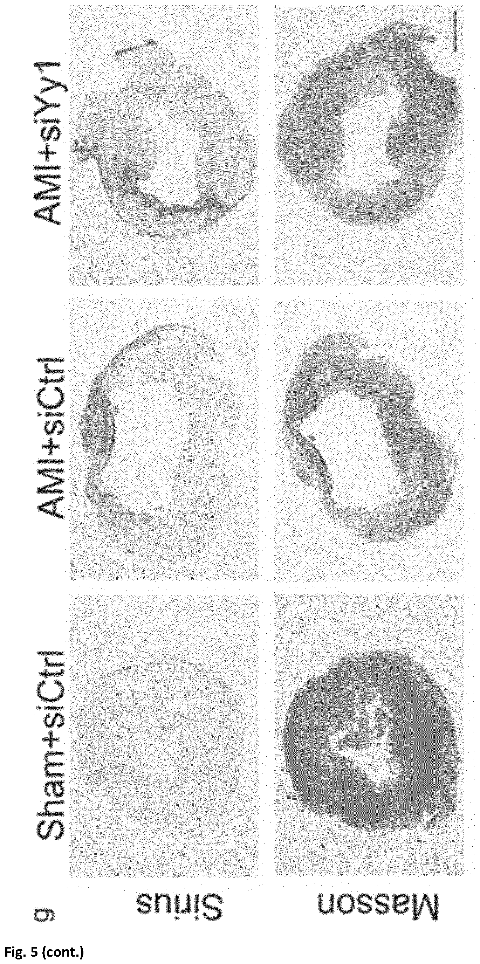

Figure 5.- siYy1 therapy improves cardiac function and AMI-induced cardiac hypertrophy. (a) Ratio between heart weight and tibia length (HW/TL) as a measure of cardiac hypertrophy. (b, c) Real-time PCR quantification of markers for cardiac remodeling in left ventricular tissue; Myh7/Myh6, ratio of mRNAs encoding β- and α-myosin heavy chain. Nppa, atrial natriuretic peptide. (d) CM area. ns: non-significative; ***P < 0.001 respect to their respective sham group. ## p<0.01, ### p<0.001, respect to AMI+siCtrl group. CM: cardiomyocyte.

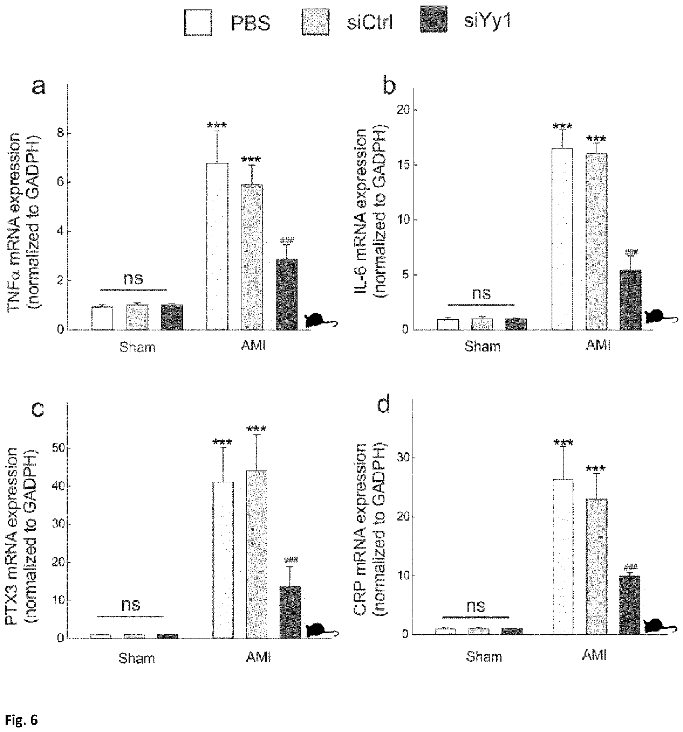

Figure 6.- siYy1 therapy prevents cardiac fibrosis following AMI. (a-f) TGF-β, smad-2, smad-3, α-sma, col1a1, and col3a1 mRNA levels; data were normalized to GAPDH mRNA levels. (g) Representative image sections from Sirius Red and Masson-stained myocardium of the indicated groups; Scale bar: 0.25 cm. ns: non-significative; ***P < 0.001 respect to their respective sham group. ### p<0.001, respect to AMI+siCtrl group.

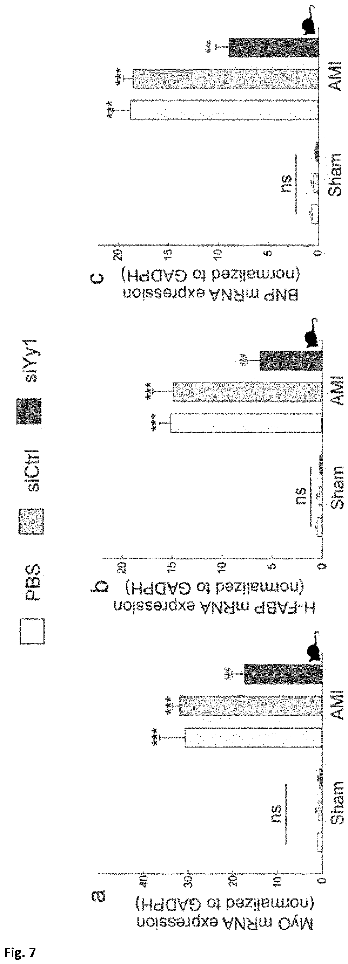

Figure 7.- siYy1 therapy blocks adverse cardiac inflammation following AMI. (a-d) TNFα, IL-6, PTX3 and CRP mRNA levels; data were normalized to GAPDH mRNA levels. ns: non-significative; ***P < 0.001 respect to their respective sham group; ### p<0.001, respect to AMI+siCtrl group. TNF: Tumoral necrosis factor; IL: interleukin; PTX: pentraxin: CRP: C-reactive protein.

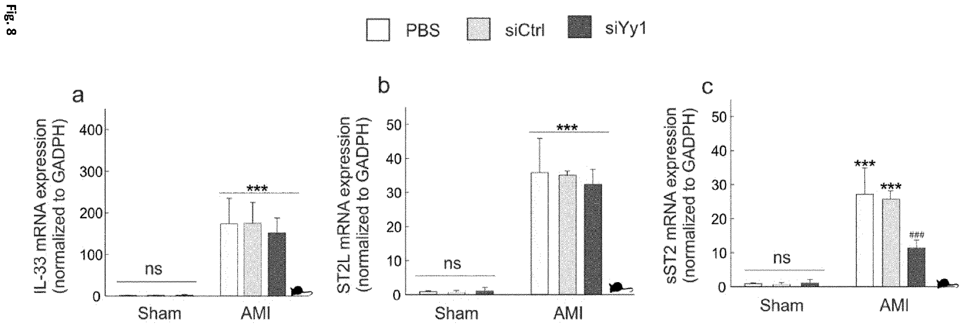

Figure 8.- siYy1 therapy improves myocardial death following AMI. (a-c) MyO, H-FABP and BNP mRNA levels; data are normalized to GAPDH mRNA levels. ***P < 0.001 respect to their respective sham group; ### p<0.001, respect to AMI+siCtrl group. MyO: myoglobin; H-FABP: Fatty acid-binding protein; BNP: brain natriuretic peptide.

### Description of the invention

The present invention relates generally to compounds which silence the endogenous Yy1 expression resulting in a decrease in the expression and release of sST2, which facilitates the cardioprotective response related to IL-33/ST2L axis; particularly to small interfering RNAs (siRNAs) that reduce Yy1 expression levels, and to the administration of these siRNAs in a subject in need thereof 2 to 48 hours after AMI, for the prevention or treatment of adverse myocardial remodeling, in particular in the prevention or treatment of cardiac complications following AMI such as myocardial fibrosis, cardiac inflammation, cardiac hypertrophy and/or functional impairment. Altogether, these findings solve the technical problem of providing an effective alternative treatment to reperfusion therapy two or more hours after the AMI.

It is herein noted that the YY1 (Yin Yang 1) is a transcriptional repressor protein in humans that is encoded by the YY1 gene (Shi Y, Seto E, Chang LS, Shenk T (October 1991). "Transcriptional repression by YY1, a human GLI-Krüppel-related protein, and relief of repression by adenovirus E1A protein". Cell. 67 (2): 377-88; Zhu W, Lossie AC, Camper SA, Gumucio DL (April 1994). "Chromosomal localization of the transcription factor YY1 in the mouse and human". Mammalian Genome. 5 (4): 234-6). More preferably, the present invention relates to compounds which down-regulate the expression of the endogenous Yy1 in cardiac cells of a human or animal subject with respect to the expression observed in the absence of the compound in said cells, such as siRNAs, preferably those listed as compound 1 having sense SEQ ID NO 1 and anti sense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8; and to the use of these compounds in the prevention or treatment of adverse myocardial remodeling following AMI, in particular in the prevention or treatment of cardiac complications following AMI such as myocardial fibrosis, cardiac inflammation, cardiac hypertrophy and/or functional impairment, wherein these compounds are administered to a subject in need thereof, 2 to 48 hours after the onset of the AMI. Preferably, such compounds are administered between 4 to 48 hours after AMI. More preferably, between 6 to 48 hours after AMI. Still more preferably, between 12 to 48 hours or between 24 to 48 hours after AMI. Still more preferably between 2, 4, 6 or 12 to 24 hours after AMI.

The invention further provides a use of a therapeutically effective dose of one or more compounds which down-regulate the expression of the endogenous Yy1, such as siRNAs, preferably those listed as compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8; for the preparation of a composition for promoting recovery in a patient suffering from adverse myocardial remodeling following AMI, in particular suffering from cardiac complications following AMI such as myocardial fibrosis, cardiac inflammation, cardiac hypertrophy and/or functional impairment, by administering said composition to a patient in need thereof within the time intervals indicated above after AMI.

The present invention thus provides methods and compositions for inhibiting expression of the endogenous Yy1 *in vivo* in cardiac cells of a human or animal subject with respect to the expression observed in the absence of the compound in said cells. In general, the method includes administering oligoribonucleotides, such as small interfering RNAs (i.e., siRNAs) that are targeted to the endogenous Yy1 in cardiac cells and hybridize to, or interact with, said mRNAs under biological conditions (within the cardiac cell), in an amount sufficient to down-regulate expression of the endogenous Yy1 by an RNA interference mechanism.

Thus, in accordance with the present invention, the siRNA molecules or inhibitors of the endogenous Yy1 may be used as drugs to prevent, treat or promote recovery in a patient in need thereof after an AMI, by administering these drugs within the time intervals indicated previously after the onset of the AMI. In particular, these drugs are administered to prevent or treat adverse myocardial remodeling following AMI, in particular to prevent or treat cardiac complications following AMI such as myocardial fibrosis, cardiac inflammation, cardiac hypertrophy and/or functional impairment. These compounds are administered to a subject in need thereof 2 to 48 hours, or in any subrange thereof, after the onset of the myocardial infarction (AMI).

The present invention thus provides double-stranded oligoribonucleotides (siRNAs), which down-regulate the expression of the endogenous Yy1 in cardiac cells of a human or animal subject with respect to the expression observed in the absence of the compound in said cells (from herein after **"Compound/s of the invention"** or **"siRNA of the invention").** An siRNA of the invention or a compound of the invention is a duplex oligoribonucleotide in which the sense strand is derived from the mRNA sequence of the endogenous Yy1, and the antisense strand is complementary to the sense strand. In general, some deviation from the target mRNA sequence is tolerated without compromising the siRNA activity (see e.g. Czauderna et al 2003 Nucleic Acids Research H(H), 2705-2716). An siRNA of the invention inhibits gene expression on a post-transcriptional level with or without destroying the mRNA. Without being bound by theory, siRNA may target the mRNA for specific cleavage and degradation and/ or may inhibit translation from the targeted message.

Generally, the siRNAs used in the present invention comprise a ribonucleic acid comprising a double stranded structure, whereby the double-stranded structure comprises a first strand and a second strand, whereby the first stand comprises a first stretch of contiguous nucleotides and whereby said first stretch is at least partially complementary to a target nucleic acid (the endogenous Yy1), and the second strand comprises a second stretch of contiguous nucleotides and whereby said second stretch is at least partially identical to a target nucleic acid (the endogenous Yy1). The strands may be modified on the sugar and /or on the phosphate and /or on the base, or alternatively may be unmodified. In one embodiment of the invention the said first strand and/or said second strand comprises a plurality of groups of modified nucleotides having a modification at the 2'-position whereby within the strand each group of modified nucleotides is flanked on one or both sides by a flanking group of nucleotides whereby the flanking nucleotides forming the flanking group of nucleotides is either an unmodified nucleotide or a nucleotide having a modification different from the modification of the modified nucleotides. Further, said first strand and/or said second strand may comprise said plurality of modified nucleotides and may comprises said plurality of groups of modified nucleotides.

The group of modified nucleotides and/or the group of flanking nucleotides may comprise a number of nucleotides whereby the number is selected from the group comprising one nucleotide to 10 nucleotides. In connection with any ranges specified herein it is to be understood that each range discloses any individual integer between the respective figures used to define the range including said two figures defining said range. In the present case the group thus comprises one nucleotide, two nucleotides, three nucleotides, four nucleotides, five nucleotides, six nucleotides, seven nucleotides, eight nucleotides, nine nucleotides and ten nucleotides.

The pattern of modified nucleotides of said first strand may be shifted by one or more nucleotides relative to the pattern of modified nucleotides of the second strand.

The modifications discussed above may be selected from the group comprising amino, fluoro, methoxy alkoxy, alkyl, amino, fluoro, chloro, bromo, CN, CF, imidazole, caboxylate, thioate, Ci to Cio lower alkyl, substituted lower alkyl, alkaryl or aralkyl, OCF3, OCN, O-, S-, or N- alkyl; O-, S-, or N-alkenyl; SOCH3; SO2CH3; ONO2; NO2, N3; heterozycloalkyl; heterozycloalkaryl; aminoalkylamino; polyalkylamino or substituted silyl, as, among others, described in European patents EP O 586 520 B1 or EP O 618 925 B1.

The double stranded structure of the siRNA may be blunt ended, on one or both sides. More specifically, the double stranded structure may be blunt ended on the double stranded structure's side which is defined by the 5'- end of the first strand and the 3'-end of the second strand, or the double stranded structure may be blunt ended on the double stranded structure's side which is defined by at the 3'-end of the first strand and the 5'∼end of the second strand.

Additionally, at least one of the two strands may have an overhang of at least one nucleotide at the 5'- end; the overhang may consist of at least one deoxyribonucleotide. At least one of the strands may also optionally have an overhang of at least one nucleotide at the 3'-end.

The length of the double-stranded structure of the siRNA is typically from about 17 to 27 and more preferably 19 or 21 bases Further, the length of said first strand and/or the length of said second strand may independently from each other be selected from the group comprising the ranges of from about 15 to about 27 bases, 17 to 21 bases and 18 or 19 bases. A particular example is 27 bases. Additionally, the complementarity between said first strand and the target nucleic acid may be perfect, or the duplex formed between the first strand and the target nucleic acid may comprise at least 15 nucleotides wherein there is one mismatch or two mismatches between said first strand and the target nucleic acid forming said double-stranded structure.

In some cases both the first strand and the second strand each comprise at least one group of modified nucleotides and at least one flanking group of nucleotides, whereby each group of modified nucleotides comprises at least one nucleotide and whereby each flanking group of nucleotides comprising at least one nucleotide with each group of modified nucleotides of the first strand being aligned with a flanking group of nucleotides on the second strand, whereby the most terminal 5' nucleotide of the first strand is a nucleotide of the group of modified nucleotides, and the most terminal 3' nucleotide of the second strand is a nucleotide of the flanking group of nucleotides. Each group of modified nucleotides may consist of a single nucleotide and/or each flanking group of nucleotides may consist of a single nucleotide.

Additionally, it is possible that on the first strand the nucleotide forming the flanking group of nucleotides is an unmodified nucleotide which is arranged in a 3' direction relative to the nucleotide forming the group of modified nucleotides, and on the second strand the nucleotide forming the group of modified nucleotides is a modified nucleotide which is arranged in 5' direction relative to the nucleotide forming the flanking group of nucleotides.

Further the first strand of the siRNA may comprise eight to twelve, preferably nine to eleven, groups of modified nucleotides, and the second strand may comprise seven to eleven, preferably eight to ten, groups of modified nucleotides.

The first strand and the second strand may be linked by a loop structure, which may be comprised of a non-nucleic acid polymer such as, inter alia, polyethylene glycol. Alternatively, the loop structure may be comprised of a nucleic acid.

Further, the 5'-terminus of the first stand of the siRNA may be linked to the 3'-terminus of the second strand, or the 3'-end of the first stand may be linked to the 5'-terminus of the second strand, said linkage being via a nucleic acid linker typically having a length between 10-2000 nucleobases.

In particular, and as already indicated previously, the invention provides a compound of the invention or an siRNA of the invention selected from the group consisting of compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8, wherein any of these compounds may optionally include any of the modifications indicated throughout the present description.

It will be thus readily understood by those skilled in the art that the compounds of the present invention consist of a plurality of nucleotides, which are linked through covalent linkages. Each such covalent linkage may be a phosphodiester linkage, a phosphothioate linkage, or a combination of both, along the length of the nucleotide sequence of the individual strand. Other possible backbone modifications are described inter alia in U.S. Patent Nos. 5,587,361; 6,242,589; 6,277,967; 6,326,358; 5,399,676; 5,489,677; and 5,596,086.

The invention further provides a vector capable of expressing any of the aforementioned oligoribonucleotides in unmodified form in a cell after which appropriate modification may be made.

The invention also provides a composition comprising one or more of the compounds of the invention or siRNAs of the invention in a carrier, preferably a pharmaceutically acceptable carrier. This composition may comprise a mixture of two or more different siRNAs, such as any of compounds 1 to 3 of the present invention.

The invention also provides a composition comprising a carrier and one or more of the compounds of the invention in an amount effective to down-regulate expression in a cell of the endogenous Yy1.

The present invention thus provides a method of prevention or treatment of adverse myocardial remodeling following AMI, in particular in the prevention or treatment of cardiac complications following AMI such as myocardial fibrosis, cardiac inflammation, cardiac hypertrophy and/or functional impairment, 2 to 48 hours after AMI in a patient, comprising administering to the patient a compound or composition described in the invention in a therapeutically effective dose so as to thereby prophylactically or therapeutically treat the patient. Preferably, the method is administered between 4 to 48 hours after AMI. More preferably, between 6 to 48 hours after AMI. Still more preferably, between 12 to 48 hours or 24 to 48 hours after AMI. Still more preferably, between 2, 4, 6 or 12 to 24 hours after AMI.

Delivery: Delivery systems aimed specifically at the enhanced and improved delivery of siRNA into mammalian cells have been developed, see, for example, Shen et al (FEBS letters 539: 111-114 (2003)), Xia et al., Nature Biotechnology 20: 1006-1010 (2002), Reich et al., Molecular Vision 9: 210-216 (2003), Sorensen et al. (J.Mol.Biol. 327: 761-766 (2003), Lewis et al., Nature Genetics 32: 107-108 (2002) and Simeoni et al., Nucleic Acids Research 31, 11 : 2717-2724 (2003). siRNA have been successfully used for inhibition in primates; for further details see Tolentino et al., Retina 24(1) February 2004 I 132-138. Respirator}' formulations for siRNA are described in U.S. patent application No. 2004/0063654 of Davis et al. Cholesterol-conjugated siRNAs (and other steroid and lipid conjugated siRNAs) can been used for delivery see Soutschek et al Nature 432: 173- 177(2004) Therapeutic silencing of an endogenous gene by systemic administration of modified siRNAs; and Lorenz et al. Bioorg. Med. Chemistry. Lett. 14:4975-4977 (2004) Steroid and lipid conjugates of siRNAs to enhance cellular uptake and gene silencing in liver cells.

The compounds, siRNAs or pharmaceutical compositions of the present invention are administered and dosed in accordance with good medical practice, taking into account the clinical condition of the individual patient, the site and method of administration, scheduling of administration, patient age, sex, body weight and other factors known to medical practitioners.

The "therapeutically effective dose" for purposes herein is thus determined by such considerations as are known in the art. The dose must be effective to achieve improvement including but not limited to improved survival rate or more rapid recovery, or improvement or elimination of symptoms and other indicators as are selected as appropriate measures by those skilled in the art. The compounds of the present invention can be administered by any of the conventional routes of administration. It should be noted that the compound can be administered as the compound or as pharmaceutically acceptable salt and can be administered alone or as an active ingredient in combination with pharmaceutically acceptable carriers, solvents, diluents, excipients, adjuvants and vehicles. The compounds can be administered orally, subcutaneously or parenterally including intravenous, intraarterial, intramuscular, intraperitoneally, and intranasal administration as well as intrathecal and infusion techniques. Preferably, the compounds, siRNAs or pharmaceutical compositions of the invention are administered parenterally, preferably by the intravenous route. Implants of the compounds are also useful. Liquid forms may be prepared for injection, the term including subcutaneous, transdermal, intravenous, intramuscular, intrathecal, and other parental routes of administration. The liquid compositions include aqueous solutions, with and without organic co-solvents, aqueous or oil suspensions, emulsions with edible oils, as well as similar pharmaceutical vehicles. In addition, under certain circumstances the compositions for use in the novel treatments of the present invention may be formed as aerosols, for intranasal and like administration. The patient being treated is a warm-blooded animal and, in particular, mammals including man. The pharmaceutically acceptable carriers, solvents, diluents, excipients, adjuvants and vehicles as well as implant earners generally refer to inert, non-toxic solid or liquid fillers, diluents or encapsulating material not reacting with the active ingredients of the invention and they include liposomes and microspheres. Examples of delivery systems useful in the present invention include U. S. Patent Nos. 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art. In one specific embodiment of this invention topical and transdermal formulations are particularly preferred.

In general, the active dose of compound for humans is in the range of from 1 ng/kg to about 20-100 mg/kg body weight per day, preferably about 0.01 mg to about 2-10 mg/kg body weight per day.

The term "treatment" as used herein refers to administration of a therapeutic substance effective to ameliorate symptoms associated with a disease, to lessen the severity or cure the disease, or to prevent the disease from occurring. In a particular embodiment, the administration comprises intravenous administration. In another particular embodiment the administration comprises topical or local administration.

Another aspect of the invention is a method of treatment in a patient of myocardial fibrosis, cardiac inflammation, cardiac hypertrophy and/or functional impairment within the time intervals indicated throughout the present specification after AMI, comprising administering to the patient a pharmaceutical composition of the invention in a therapeutically effective amount so as to thereby prophylactically or therapeutically treat the patient.

In another aspect of the invention a pharmaceutical composition is provided which comprises any of compounds 1 to 3 or vectors which express these oligonucleotides and a pharmaceutically acceptable carrier. Another aspect of the invention is the use of a therapeutically effective amount of any of the above oligoribonucleotides or vectors for the preparation of a medicament for promoting recovery in a patient suffering from myocardial fibrosis, cardiac inflammation, cardiac hypertrophy and/or functional impairment within the time intervals indicated throughout the present specification after AMI.

The present invention also provides for a process of preparing a pharmaceutical composition, which comprises admixing one or more compounds of the present invention with a pharmaceutically acceptable carrier.

In a preferred embodiment, the compound used in the preparation of a pharmaceutical composition is admixed with a carrier in a pharmaceutically effective dose. In a particular embodiment the compound of the present invention is conjugated to a steroid or to a lipid or to another suitable molecule e.g. to cholesterol.

Modifications or analogs of nucleotides can be introduced to improve the therapeutic properties of the nucleotides. Improved properties include increased nuclease resistance and/or increased ability to permeate cell membranes.

Accordingly, the present invention also includes all analogs of, or modifications to, a oligonucleotide of the invention that does not substantially affect the function of the polynucleotide or oligonucleotide. In a preferred embodiment such modification is related to the base moiety of the nucleotide, to the sugar moiety of the nucleotide and/or to the phosphate moiety of the nucleotide.

In embodiments of the invention, the nucleotides can be selected from naturally occurring or synthetically modified bases. Naturally occurring bases include adenine, guanine, cytosine, thymine and uracil. Modified bases of the oligonucleotides include inosine, xanthine, hypoxanthine, 2- aminoadenine, 6-methyl-, 2-propyl- and other alkyl- adenines, 5-halo uracil, 5-halo cytosine, 6-aza cytosine and 6-aza thymine, pseudo uracil, 4-thiuracil, 8-halo adenine, 8-aminoadenine, 8-thiol adenine, 8-thiolalkyl adenines, 8-hydroxyl adenine and other 8-substituted adenines, 8-halo guanines, 8-arnino guanine, 8-thiol guanine, 8-thioalkyl guanines, 8-hydroxyl guanine and other substituted guanines, other aza and deaza adenines, other aza and deaza guanines, 5-trifluoromethyl uracil and 5- trifluoro cytosine.

In addition, analogs of nucleotides can be prepared wherein the structures of the nucleotides are fundamentally altered and are better suited as therapeutic or experimental reagents. An example of a nucleotide analog is a peptide nucleic acid (PNA) wherein the deoxyribose (or ribose) phosphate backbone in DNA (or RNA) is replaced with a polyamide backbone similar to that found in peptides. PNA analogs have been shown to be resistant to degradation by enzymes and to have extended lives in vivo and in vitro. Further, PNAs have been shown to bind more strongly to a complementary DNA sequence than to a DNA molecule. This observation is attributed to the lack of charge repulsion between the PNA strand and the DNA strand. Other modifications that can be made to oligonucleotides include polymer backbones, cyclic backbones, or acyclic backbones.

In one embodiment the modification is a modification of the phosphate moiety, whereby the modified phosphate moiety is selected from the group comprising phosphothioate.

The compounds of the present invention can be synthesized by any of the methods that are well-known in the art for synthesis of ribonucleic (or deoxyribonucleic) oligonucleotides. Such synthesis is, among others, described in Beaucage S.L. and Iyer R.P., Tetrahedron 1992; 48: 2223-2311, Beaucage SX. and Iyer R.P., Tetrahedron 1993; 49: 6123-6194 and Caruthers M.H. et al., Methods Enzymol. 1987; 154: 287-313, the synthesis of thioates is, among others, described in Eckstein F., Annu. Rev. Biochem. 1985; 54: 367-402, the synthesis ofRNA molecules is described in Sproat B., in Humana Press 2005 Edited by Herdewijn P.; Kap. 2: 17-31 and respective downstream processes are, among others, described in Pingoud A. et. al., in IRL Press 1989 Edited by Oliver R.W.A.; Kap. 7: 183-208 and Sproat B., in Humana Press 2005 Edited by Herdewijn P.; Kap. 2: 17-31 (supra).

Other synthetic procedures are known in the art e.g. the procedures as described in Usman et al., 1987, J. Am. Chem. Soc, 109, 7845; Scaringe et al., 1990, Nucleic Acids Res., 18, 5433; Wincott et al., 1995, Nucleic Acids Res. 23, 2677-2684; and Wincott et al., 1997, Methods Mol. Bio., 74, 59, and these procedures may make use of common nucleic acid protecting and coupling groups, such as dimethoxytrityl at the 5'-end, and phosphoramidites at the 3 -end. The modified (e.g. 2'-O-methylated) nucleotides and unmodified nucleotides are incorporated as desired.

The oligonucleotides of the present invention can be synthesized separately and joined together post- synthetically, for example, by ligation (Moore et al., 1992, Science 256, 9923; Draper et al., International PCT publication No. WO93/23569; Shabarova et al., 1991, Nucleic Acids Research 19, 4247; Bellon et al., 1997, Nucleosides Sc Nucleotides, 16, 951; Bellon et al., 1997, Bioconjugate Chem. 8, 204), or by hybridization following synthesis and/or deprotection.

It is noted that a commercially available machine (available, inter alia, from Applied Biosystems) can be used; the oligonucleotides are prepared according to the sequences disclosed herein. Overlapping pairs of chemically synthesized fragments can be ligated using methods well known in the art (e.g., see U.S. Patent No. 6,121,426). The strands are synthesized separately and then are annealed to each other in the tube. Then, the double-stranded siRNAs are separated from the single-stranded oligonucleotides that were not annealed (e.g. because of the excess of one of them) by HPLC. In relation to the siRNAs or siRNA fragments of the present invention, two or more such sequences can be synthesized and linked together for use in the present invention.

The compounds of the invention can also be synthesized via a tandem synthesis methodology, as described in US patent application publication No. US2004/0019001 (McSwiggen), wherein both siRNA strands are synthesized as a single contiguous oligonucleotide fragment or strand separated by a cleavable linker which is subsequently cleaved to provide separate siRNA fragments or strands that hybridize and permit purification of the siRNA duplex. The linker can be a polynucleotide linker or a non-nucleotide linker.

The compounds of the present invention can be delivered either directly or with viral or non-viral vectors. When delivered directly the sequences are generally rendered nuclease resistant. Alternatively the sequences can be incorporated into expression cassettes or constructs such that the sequence is expressed in the cell as discussed herein below. Generally the construct contains the proper regulatory sequence or promoter to allow the sequence to be expressed in the targeted cell. Vectors optionally used for delivery of the compounds of the present invention are commercially available, and may be modified for the purpose of delivery of the compounds of the present invention by methods known to one of skill in the art.

The following examples illustrate but do not limit the present invention.

### Examples

### MATERIAL AND METHODS

*Animal Care and ethical aspects.* C57Bl/6J male mice (weighing 25-30 g) were purchased from the Jackson Laboratory. Mice were housed in specific pathogen free environment with a relative humidity of 50 ± 5% at 23 ± 2 °C with 12 h light and dark cycles. Mice had free access to food and water. All animal experiments were approved by the Ethics Review committee for animal use at the University of Murcia (Permit number: A13150105). After 7 days' adaptation, the animals were subjected to a surgical procedure to induce AMI before systemic Yy1 genetic silencing. Animals were randomly split into six groups (see groups and experimental design).

*Induction of experimental AMI in mice.* Before the surgical procedures, animals were anesthetized with intraperitoneal ketamine (75 mg/kg) and medetomidine (0.5 mg/kg), before being intubated and ventilated with a 18 gauge intravenous catheter and placed in supine position over a temperature control pad. The animals were monitored by electrocardiogram (ECG) electrodes connected to the limbs through small needles inserted subcutaneously. Left-sided thoracotomy was performed by a small incision between the third and fourth intercostal spaces. The incision was expanded by a blunt ended retractor in such a manner that the lungs were avoided in the area of retraction. The pericardial sac surrounding the heart was cut open, but the heart was not exteriorized. The ligation site of the left anterior descending coronary artery (LAD) was determined 4 mm away from the origin. Using a tapered atraumatic needle, a 8-0 silk ligature was passed underneath the LAD and tied with three knots. Visible blanching and cyanosis of the anterior wall of the left ventricle and swelling of the left atrium were taken as indicative of successful ligation. The procedure was considered successful if the ECG showed ST-segment elevation and the anterior wall of the left ventricle became blanched. Ribs and muscles were closed using 6-0 vicryl dissolvable sutures, leaving a small gap to aspirate any air left in the chest cavity. The air was aspirated by an in-house tube (2 mm diameter), again without touching the lungs. At the time of closure, neomycin powder and betadine were applied to the muscle and skin stitch sites, respectively. The surgical site was dressed daily to avoid any infection and to monitor for any dehiscence of the suture site. The entire procedure was performed within 20 min of the induction of anesthesia. Sham-operated rats underwent the same procedure without any ligation. After surgery, the animals received four doses of buprenorphine (0.05 mg/kg, subcutaneous) at 8 h intervals. The sham groups underwent the same surgical procedure except that the LAD coronary artery was not occluded. Electrocardiographic monitoring was used to confirm ST segment elevation after AMI induction. To evaluate the evolution of cardiac damage, an echocardiographic study was performed on each mouse before surgery, and after 24 h, 1 and 4 weeks following AMI(16).

*Experimental design and study protocol.* At 24 post-AMI, survived animals were randomize rinsed into six experimental groups: (1) Sham group treated with PBS by intravenous route (Sham group PBS, n=10); (2) Sham group treated with siControl (siCtrl, see table 3) by intravenous route (Sham groups siCtrl, n=10); (3) Sham group treated with siYy1 (the term "siYy1" shall be understood herein a a pool formed by four specific siRNA sequences to silent the endogenous Yy1 levels (compounds 1 to 4 of Table 1) by intravenous route (Sham group siYy1, n=10); (4) Infarcted group treated with placebo (PBS) by intravenous route (AMI group PBS, n=10); (5) Infracted group treated with siControl (siCtrl) by intravenous route (AMI group siCtrl, n=10); (6) Infracted group treated with siYy1 by intravenous route (AMI group siYy1, n=10) The animals were kept in the conditions described above until their sacrifice (4 weeks after AMI).

*Endogenous Yin-Yang 1 transcription factor silencing in mice.* To generate an experimental model where the endogenous Yy1 factor expression levels are silence, mice were transfected with a mixture of 4 specific interference RNA sequences.

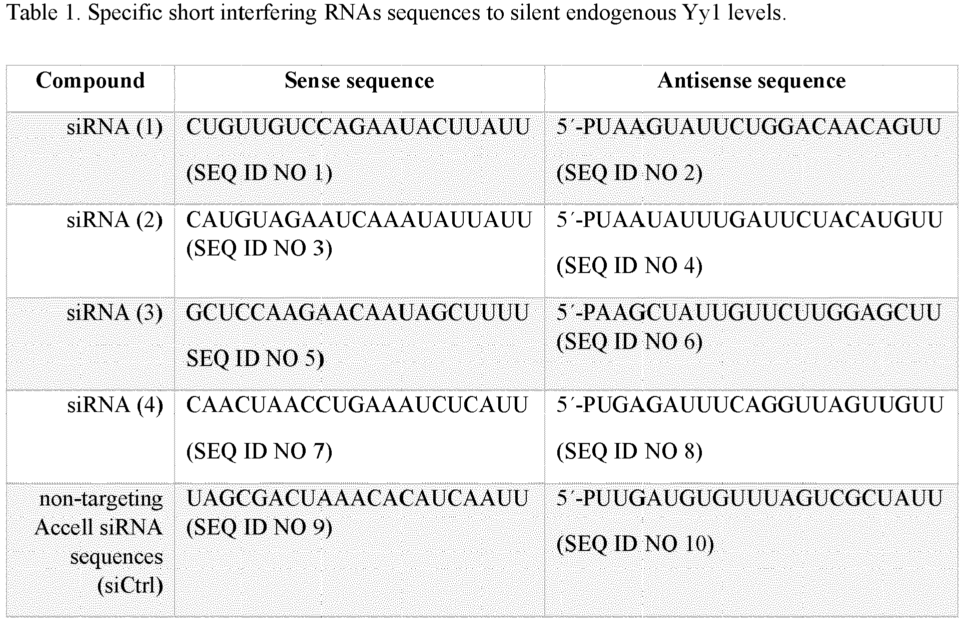

**The infusion was made intravenously, through the tail vein of the animal, after 24 h of AMI.** The siRNA sequences against Yy1 transcription factor (Custom siRNA, in vivo HPLC Accell) were acquired from Dharmacon (A-050273-13, -14, -15 and -16) and administered together in three independent doses of 3 mg/kg at days 1, 7 and 14 after AMI, that leads to a cumulative final concentration of 9 mg/kg. An outline of the experimental procedure is shown in Figure 2a. Briefly, just prior to use 75 µg of each one of the four siRNAs were mixed using Accell siRNA Delivery Media from GE Healthcare (B-005000). The target sequences both for the mouse-specific Yy1 transcription factor as well as a non-targeting Accell siRNA sequences (siCtrl), is shown in Table 1 above.

*LV structure and function.* Transthoracic echocardiography (2D and M-mode echocardiography) was performed on anaesthetized mice (1.8% isoflurane, inhalation) using a Vevo machine and a 13-MHz probe (MJFP), prior to surgery (baseline), 24 h post-AMI and 4 weeks post-AMI. From the four-chamber long axis views, the end-systolic (ESLVV) and end-diastolic left ventricular (EDLVV) volumes were determined by the Simpson method and the ejection fraction (EF) was determined automatically as EF (%) = EDLVV-ESLVV/EDLVV X 100. At the level of the chordae tendineae of the mitral valve, left ventricular end-diastolic (EDLVD) and end-systolic (ESLVD) dimension measurements were made by M-mode. Data are listed in the additional file (Table 2). The investigators who analyzed the data were blinded to the experimental groups.

**Table 2. Echocardiography measurements of sham and AMI control and siYy1 treated mice at 4 weeks post-AMI**

| Echocardiography parameters obtained from study | | | | | | |
|---|---|---|---|---|---|---|
| Treatment | Sham +PBS | Sham +siCtrl | Sham +siYy1 | AMI +PBS | AMI +siCtrl | AMI +siYy1 |
| N number | 12 | 10 | 10 | 10 | 10 | 9 |
| BW (g) | 24.03±0.13 | 24.1±0.32 | 24.62±0.41 | 25.1±0.12 | 25.20±0.15 | 25.20±0.14 |
| HR, bpm | 457.08±8.32 | 452±6.96 | 458.3±10.01 | 447.8±9.69 | 445.36±8.09 | 463±4.63*** |
| LVEDD (mm) | 4.82±0.08 | 4.61±0.15 | 4.89±0.12 | 4.74±0.54 | 4.81±0.04 | 4.43±0.22* |
| LVESD (mm) | 2.64±0.06 | 2.59±0.09 | 2.76±0.08 | 3.29±0.17 | 3.38±0.06### | 2.63±0.04*** |
| LVEDV (mL) | 71.01±0.35 | 71.15±0.41 | 71.41±3.31 | 110.22±3.54 | 105.45±5.22### | 93.53±3.46*** |
| LVESV (mL) | 35.12±0.74 | 36.14±0.52 | 31.87±0.33 | 68.58±0.69 | 68.45±0.88## | 51.45±3.33*** |
| EF (%) | 50.54±0.98 | 49.20±0.67 | 55.37±0.96 | 37.78±2.10 | 35.08±1.39### | 44.99±0.86*** |
| FS (%) | 45.22±1.25 | 43.82±0.65 | 43.56±1.54 | 30.59±2.32 | 29.73±1.56### | 40.63±1.56*** |

| | | | | | | |
|---|---|---|---|---|---|---|
| BW, body weight; HR, heart rate; LV, left ventricle; LVEDV, left ventricular end-diastolic volume; LVESV, left ventricular end-systolic volume; LVEDD, left ventricular dimensions at end diastole; LVESD, left ventricular dimensions at end systole; FS, fractional shortening; EF, ejection fraction. ###P<0.001 versus Sham+siCtrl, *P<0.05 and *** p<0.001 versus AMI+siCtrl. P values were calculated using unpaired, two-tailed Student's t-tests. Data represent means ± SEM. | | | | | | |

### RNA extraction and quantitative RT-PCR

Fresh infracted ventricle (∼ 20 mg) were washed with cold DPBS. Then, samples were placed in a pre-chilled glass Petri dish and chopped in an ice bath using sharp scissors. RNA isolation and quantitative real-time PCR were performed according to the manufacturer's protocol with minor modifications(15). The primer sequences used for quantitative real-time PCR analysis are described in table 3.

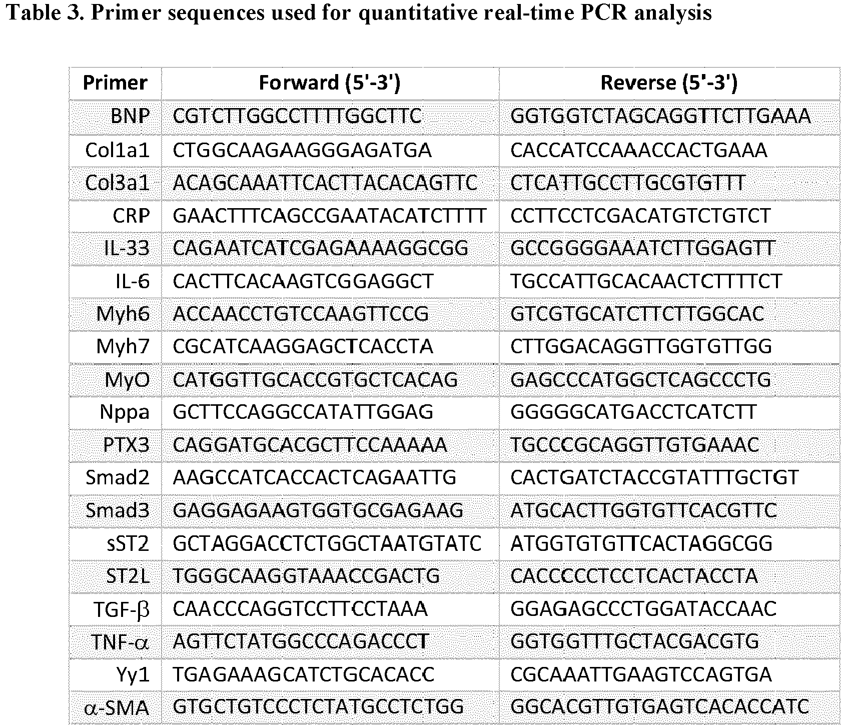

### Statistical analysis.

Data were expressed as mean ± standard error of the mean. Normality was tested using the Kolmogorov-Smirnov test. Differences between all groups were tested with a Kruskal-Wallis test. For multiple comparisons with the sham group, the Siegel-Castellan test was used. Non-parametric correlations were studied only in infarcted animals following Kendall's method. Statistical significance was assumed at p<0.05. Data were statistically analyzed using SPSS statistics 22 (IBM Corp., Armonk, NY, USA). Graphing was performed using SigmaPlot 11.0 software. P values of < 0.05 were considered statistically significant.

### Results

### AMI induces an up-regulation of Yin yang-1 (Yyl) transcription factor

First, we quantified Yy1 levels in total RNA extracted from left ventricle (LV) of infarct and non-infarct mice (Figure 1). As shown in Figure 1a, Yy1 mRNA levels in the LV infarct area increased significantly **after one week from AMI** (p<0.01); an increase that was higher when the time period was prolonged to 4 weeks (p<0.001) (Figure 1a). The increase on Yy1 mRNA did not occur in the remote LV area (Figure 1b).

### Genetic deficiency of Yy1 transcription factor prevents adverse cardiac remodeling following AMI

Next, starting from the observation that decreased Yy1 levels and activity by specific siRNAs, prevented hypertrophy of neonatal cardiomyocytes *in vitro*(15), we asked whether a global genetic deletion of Yy1 prevents the pathologic cardiac remodeling following AMI. As shown in figure 2a, and as described above, siYy1 therapy was initiated after AMI induction; immediately after ST segment elevation. When we evaluated Yy1 mRNA levels in the infarcted LV area (AMI+siCtrl group), as compared to mRNA levels obtained in sham group (Sham+siCtrl group), we observed a significant increase (p<0.001) that was prevented when animals were treated with siYy1 (AMI+siYy1 vs. AMI+siCtrl; p<0.001) (Figure 2b).

Importantly, infarcted mice treated with siYy1 were protected from AMI-induced cardiac hypertrophy and functional impairment. Treated animals showed better heart function (Figure 3) (p<0.001), lower macroscopic cardiac hypertrophy (i.e., heart weight) (p<0.001) (Figure 4a), lower levels of hypertrophic-associated marker genes Myh7 (in relation to Myh6) and Nppa (p<0.001, in both cases) (Figure 4, b-c), and lower cardiac myocytes hypertrophy (p<0.01) (Figure 4d).

Remarkably, also myocardial fibrosis was reduced in these siYy1-deficient mice, as determined by a fibrosis-associated marker genes deregulation (Figure 5, a-f). Sirius Red and Masson staining confirmed these protective results (Figure 5g).

Similar effects were obtained when we evaluated the effect of siYy1 therapy on cardiac inflammation following AMI. Again, siYy1 therapy prevented adverse inflammation following AMI in term of an inflammation-associated marker genes deregulation (Figure 6).

Next, we carried out experiments related to myocardial death. As shown in figure 7, AMI induces cell death of infarcted LV myocardium, which is characterized by a significant increase in the levels of MyO (p<0.001), H-FABP (p<0.001) and BNP mRNA levels (p<0.001) (Figure 7). siYy1 therapy prevented this increase (p<0.001, in all cases).

***The protective cardiac effect of siYy1 therapy is related to the modulation of IL-33*/*ST2 axis.***

Next, to confirm whether the beneficial effect of siYy1 therapy during cardiac remodeling *in vivo* is related with IL33/ST2L axis, the mRNA levels of IL-33, ST2L and sST2 we evaluated in the infracted LV area from mice by quantitative RT-PCR (Figure 8). AMI was associated with an increase in IL-33 (p<0.001), ST2L (p<0.001) and sST2 (p<0.01) mRNA expression. Interestingly, the lack of endogenous Yy1 had no effect on IL-33 and ST2L mRNA levels and prevented the up-regulation of myocardial sST2 mRNA level. The silencing of Yy1 in the absence of damage or the use of PBS, had no effect on IL33, ST2L and sST2 expression compared to their respective controls.

## Claims

1. A composition comprising a compound capable of reducing the expression of the Yin Yang 1 (Yyl) gene in cardiac cells of a human or animal subject with respect to the expression observed in the absence of the compound in said cells, wherein the compound is an interference RNA (RNAi) of the Yy1 gene, and wherein said compound is for use in prevention or treatment of adverse myocardial remodeling following myocardial infarction (AMI) in the subject, and wherein said composition is administered 2 to 48 hours after the onset of myocardial infarction (AMI) in the subject.

2. The composition for use according to claim 1, wherein the compound is administered 4 to 48 hours after myocardial infarction (AMI).

3. The composition for use according to claim 1, wherein the compound is administered 6 to 48 hours after myocardial infarction (AMI).

4. The composition for use according to claim 1, wherein the compound is administered 12 to 48 hours after myocardial infarction (AMI).

5. The composition for use according to claim 1, wherein the compound is administered 24 to 48 hours after myocardial infarction (AMI).

6. The composition for use according to any of claims 1 to 5, wherein said compound capable of reducing the expression of the Yin Yang 1 (Yyl) gene in cardiac cells of a human or animal subject with respect to the expression observed in the absence of the compound in said cells, is an interference RNA (RNAi) of the Yy1 gene.

7. The composition for use according to any of claims 6 or 7, wherein the siRNA is selected from the list consisting of any of the following compounds: compound 1 having sense SEQ ID NO 1 and antisense SEQ ID NO 2, compound 2 having sense SEQ ID NO 3 and antisense SEQ ID NO 4, compound 3 having sense SEQ ID NO 5 and antisense SEQ ID NO 6, and compound 4 having sense SEQ ID NO 7 and antisense SEQ ID NO 8.

8. The composition for use according to any of claims 6 or 7, wherein the compound is administered intravenously.

9. The composition for use according to any of claims 6 to 8, wherein the composition is a pharmaceutical composition comprising a therapeutically effective amount of an siRNA as defined in of claims 6 or 7 or vectors which express these oligonucleotides and a pharmaceutically acceptable carrier.
